# EUROPEAN PATENT APPLICATION

(11) **EP 3 772 628 A1**
(43) Date of publication of application: **10.02.2021**
(21) Application number: 20188577.9
(22) Date of filing: 30.07.2020
(51) Int. Cl.: F26B 3/04, F26B 9/00, F26B 21/10

(54) **DRYER AND SANITIZER FOR RECHARGEABLE ELECTRONIC DEVICES**

(30) Priority: 07.08.2019 US 201962883761 P; 28.10.2019 US 201916665045; 14.01.2020 US 202016742156; 21.04.2020 US 202016853822
(71) Applicant: Ear Technology Corporation, Johnson City TN 37601 (US)
(72) Inventor: SCHUMAIER, Daniel R., Elizabethton, TN 37643 (US); HARRISON, Karlee, Johnson City, TN 37615 (US)
(74) Representative: Nordic Patent Service A/S

(57) **Abstract**

A dryer and sanitizer unit for rechargeable electronic devices includes a drying chamber formed by a base attached to a housing and a repositionable lid that covers the base. The drying chamber is configured to enclose one or more rechargeable electronic devices and one or more electronic charger units associated with the one or more electronic devices. The drying chamber includes one or more passages through which electric cords associated with the one or more electronic charger units may pass out of the drying chamber. An air inlet is provided in the housing in gas flow communication with the drying chamber and a filter is associated with the air inlet. One or more disinfecting light sources located within the drying chamber.

## Description

### FIELD

This relates to a dryer and sanitizer unit configured for drying and sanitizing a wide variety of rechargeable electronic devices including, but not limited to, cell phones, hearing aids, watches, earphones, disclosure relates to devices for drying and sanitizing electronic devices. More particularly, this disclosure headphones, and the like, and their chargers which come in many different sizes and shapes from different manufacturers.

### BACKGROUND

Improvement is desired in devices for charging, drying and sanitizing electronic devices. During use, the electronic devices such as cell phones, hearing aids, earphones, headphones, watches, and the like may collect bacteria and gather moisture that can be harmful to the electronic devices. The batteries of the electronic devices also discharge during use and require recharging.

What is desired is a convenient way to recharge the electronic devices while also sanitizing and drying the electronic devices at the same time.

The present disclosure advantageously provides an easy-to-use unit that enables the charging of electronic devices while the electronic devices are sanitized and dried.

### SUMMARY

The above and other needs are met by a dryer and sanitizer unit for rechargeable electronic devices.

In one aspect, a dryer and sanitizer unit according to the disclosure includes a drying chamber formed by a base attached to a housing and a repositionable lid that covers the base. The drying chamber is configured to enclose one or more rechargeable electronic devices and one or more electronic charger units associated with the one or more electronic devices. The drying chamber includes one or more passages through which electric cords associated with the one or more electronic charger units may pass out of the drying chamber; an air inlet in the housing in gas flow communication with the drying chamber; a filter associated with the air inlet; and one or more disinfecting light sources located within the drying chamber.

In another aspect, a dryer and sanitizer unit according to the disclosure includes a base attached to a housing and a lid that together define a drying chamber that encloses one or more rechargeable electronic devices and one or more charger units associated with the one or more rechargeable electronic devices.

The unit also includes one or more passages through which electric cords associated with the one or more charger units pass out of the drying chamber; one or more disinfecting light sources located within the drying chamber; an air inlet in the housing in gas flow communication with the drying chamber; a filter associated with the air inlet; a heater configured to provide heated dry air; a fan configured to circulate the heated dry air in the drying chamber; a thermostat configured to measure air temperature in the drying chamber; and a controller in electrical communication with the thermostat and the heater. The controller maintains the air temperature in the drying chamber above a predetermined drying temperature and below a predetermined upper temperature corresponding to a temperature above which rechargeable batteries associated with the rechargeable electronic devices are vulnerable to overheating.

In a further aspect, a dryer and sanitizer unit according to the disclosure includes a base attached to a housing and a lid that together define a drying chamber that is configured to enclose one or more rechargeable electronic devices locatable within the drying chamber; one or more disinfecting light sources located within the drying chamber; an air inlet in the housing in gas flow communication with the drying chamber; a filter associated with the air inlet; a heater configured to provide heated dry air; a fan configured to circulate the heated dry air in the drying chamber; a thermostat configured to measure air temperature in the drying chamber; and a controller in electrical communication with the thermostat and the heater. The controller maintains the air temperature in the drying chamber above a predetermined drying temperature and below a predetermined upper temperature corresponding to a temperature above which rechargeable batteries associated with the rechargeable electronic devices are vulnerable to overheating.

In another aspect, the dryer and sanitizer unit according to the disclosure includes a thermostat that is operably associated with the drying chamber for measuring air temperature in the drying chamber.

In a further aspect, the dryer and sanitizer unit according to the disclosure is configured for drying and sanitizing rechargeable electronic devices selected from cell phones, watches, headphones, earphones, and other portable electronic devices.

In one aspect, the dryer and sanitizer unit according to the disclosure includes a fan and a heater in flow communication with the air inlet in the housing and holes in the base for introduction of a flow of warm air into the drying chamber.

In another aspect, the dryer and sanitizer unit according to the disclosure includes vents located in the lid to promote the flow of warm air over the rechargeable electronic devices and to provide a path for the warm air and moisture to exit the drying chamber.

In one aspect, the dryer and sanitizer unit according to the disclosure includes one or more disinfecting light sources that are operated for a predetermined time sufficient to disinfect the rechargeable electronic devices within the drying chamber, after which the heater and fan are operated for a predetermined drying cycle to introduce the warm air into the drying chamber with or without the disinfecting light sources being operated.

In further aspect, the lid of the dryer and sanitizer unit according to the disclosure has an interior surface that is UV-reflective.

In another aspect, the dryer and sanitizer unit according to the disclosure has a lid open detection circuit that is operably associated with both the lid and the disinfecting light sources and operable to turn off the disinfecting light sources if the lid is opened during operation of the disinfecting light sources.

In one aspect, the dryer and sanitizer unit according to the disclosure has a wireless nearfield proximity charger built into the base.

In one aspect, the filter for the dryer and sanitizer unit according to the disclosure is selected from a high-efficiency particulate air (HEPA) filter, and a filter material that may be impregnated with an essential oil.

### BRIEF DESCRIPTION OF THE DRAWINGS

Further advantages of the disclosure are apparent by reference to the detailed description when considered in conjunction with the figures, which are not to scale so as to more clearly show the details, wherein like reference numbers indicate like elements throughout the several views, and wherein:
FIG. 1 is a perspective view of an embodiment of a dryer and sanitizing unit according to the disclosure.
FIG. 2 is a perspective view of the dryer and sanitizing unit of FIG. 1 with the lid open and a cell phone and a charger for a cell phone installed.
FIG. 3 is a perspective view of the dryer and sanitizing unit of FIG. 1 with the lid open and a cell phone disposed on the base and attached to a charging cable.
FIG. 4 is a rear elevational view of the dryer and sanitizing unit of FIG. 1 showing an opening for a power cord and power connections to the unit.
FIG. 5 is a rear elevational view of a dryer and sanitizing unit of FIG. 1 showing a filter unit and filter attached to the housing.
FIG. 6 is a partial perspective rear view of the dryer and sanitizing unit of FIG. 5.
FIG. 7 is perspective view of a dryer and sanitizing unit according to another embodiment of the disclosure with the lid open to show a built-in wireless nearfield proximity charger in the base.
FIG. 8 is a cross-sectional view not to scale of the dryer and sanitizing unit according to FIG. 1.
FIG. 9 is a functional block diagram of electrical components of an embodiment of the dryer and sanitizer unit according to the disclosure.
FIG. 10 is a perspective view showing air flow through the dryer and sanitizer unit according to an embodiment of the disclosure.
FIG. 11 is a schematic diagram of an embodiment of an electrical circuit for use with the dryer and sanitizer unit according to the disclosure.

### DETAILED DESCRIPTION

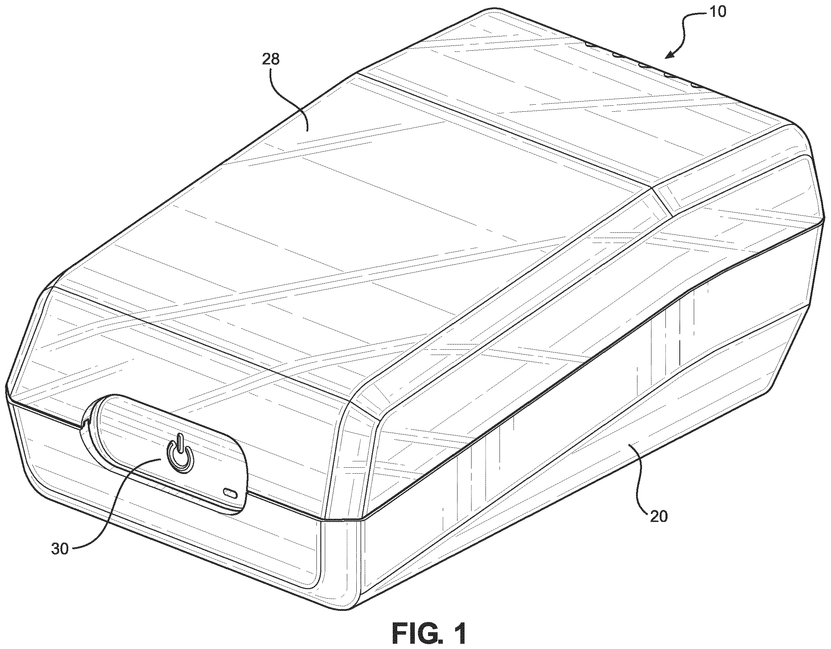

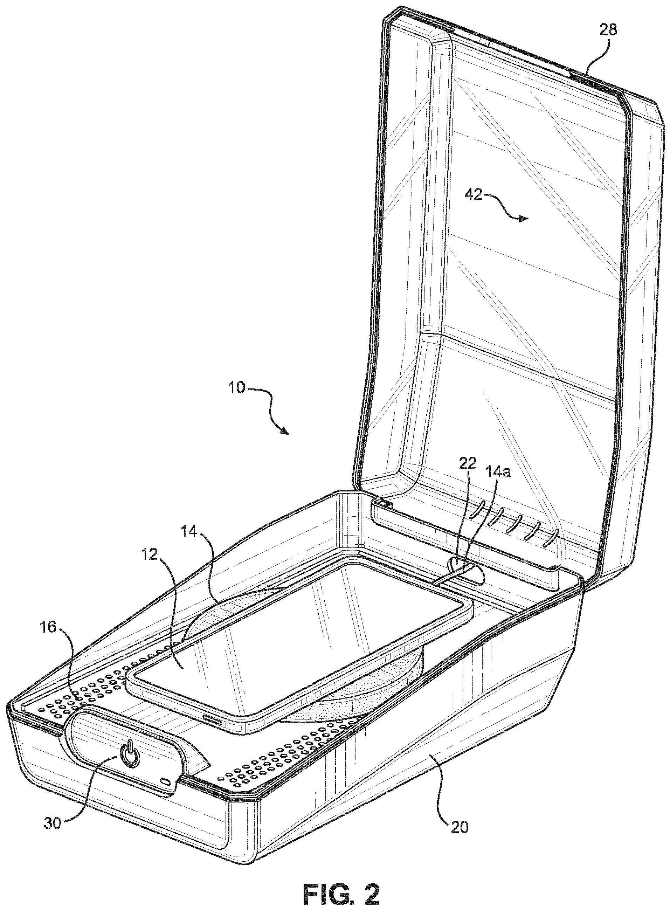

With initial reference to FIGS. 1-4 there is shown a preferred embodiment of a dryer and sanitizer unit 10 according to the disclosure configured for drying and sanitizing rechargeable electronic devices, such as portable devices in the nature of cell phones 12. The electronic devices may be disposed on a charger 14 having an electric cord 14a. The electronic devices 12 may be selected from cell phones, hearing aids, watches, headphones, earphones, and other portable rechargeable electronic devices. In some embodiments, the charger 14 may be a wireless nearfield proximity charger 14 as shown in FIG. 2.

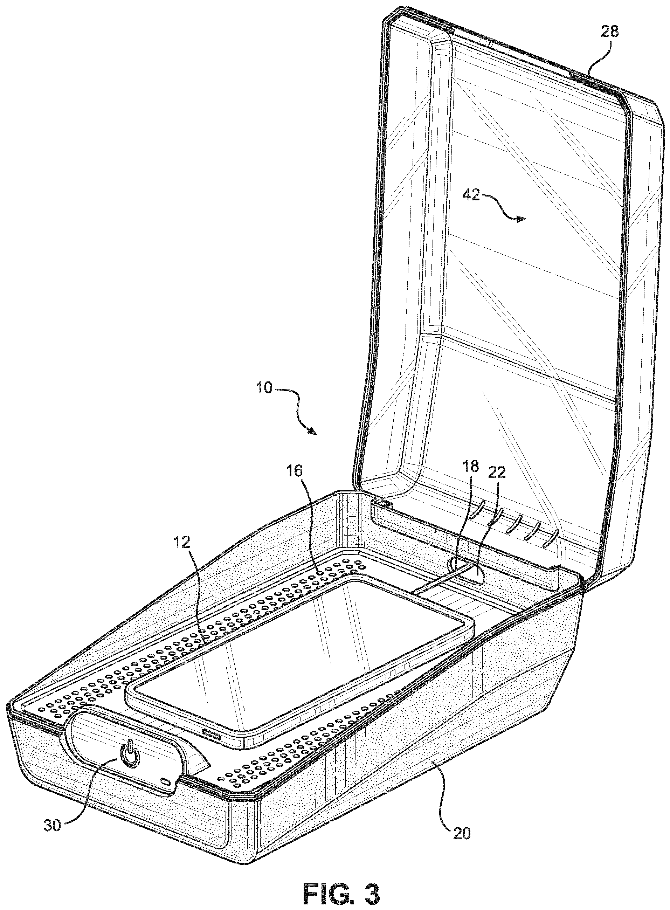

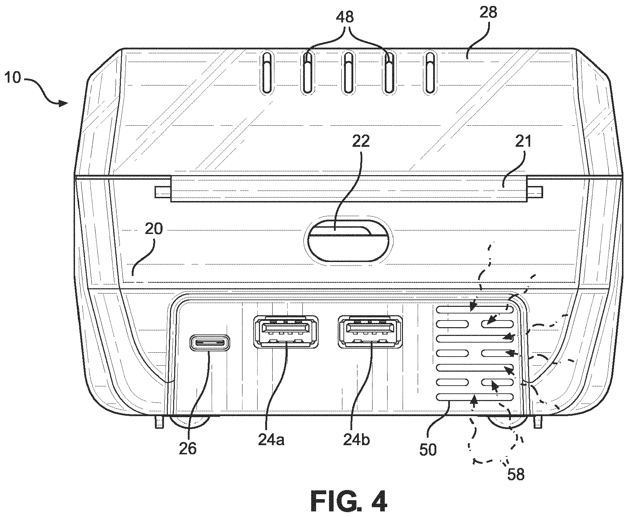

In FIG. 3, the rechargeable electronic device, such as a cell phone 12 is placed on a base 16 of the drying and sanitizing unit 10 rather than on the charger 14. In this embodiment, a charging cable 18 is directly connected to the cell phone 12 rather than to the proximity charger 14. Accordingly, the dryer and sanitizing unit 10 includes a housing 20 for the base 16 having an aperture 22 therein for the cables 14a and 18. Cables 14a and 18 may be standard USB cables for connection to USB ports 24a and 24b on the housing 20 (FIG. 4). The USB ports 24a and 24b may be Type -A USB ports that enables the charging of two different products. The USB ports 24a and 24b may provide 5 watts to 15 watts of power to the charger and/or rechargeable electronic device.

Power may be input to the dryer and sanitizing unit 10 by means of a power input 26. Power may be provided by a standard electrical plug, a USB micro plug or USB-C plug input connected to a power source, or a solar energy input.

In this regard, initially the rechargeable electronic devices are disinfected by application of sanitizing illumination. Following or simultaneous with the sanitization, heated dry air is flowed over the rechargeable electronic devices to remove moisture. The sanitization and drying operations are described in more detail below.

A lid 28 is hingedly connected to the housing 20 by hinge 21 to provide a closed chamber between the lid 28 and the housing 20 as shown in FIG. 1. A capacitive touch switch 30 is provided on the housing 20 to activate the dryer and sanitizing unit 10. The switch 30 may be operated to provide sanitization by touching the switch once, and to provide sanitization and drying by touching the switch twice. Touching the switch three times may be used to activate only the drying system.

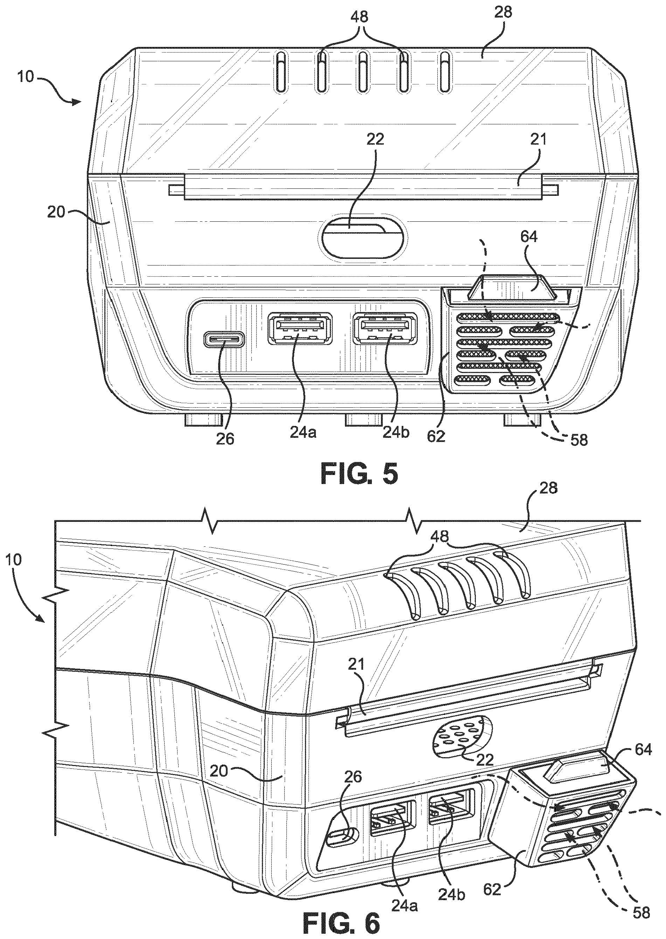

FIGs. 5 and 6 illustrate an alternative embodiment, wherein the housing includes a filter housing 62 and removable filter unit 64 disposed in the filter housing 62. The removable filter unit 64 may be a high-efficiency particulate air (HEPA) filter. In another embodiment, the removable filter may be impregnated with a sanitizing solution to further sanitize the air that is used to dry the portable electronic devices. The sanitizing solution may be an essential oil or other sanitizing solutions. Other sanitizing solutions that may be used in liquid or vapor form include, but are not limited to, a 62% by volume ethyl alcohol solution, a 6 vol. % sodium hypochlorite solution in water, white vinegar, hydrogen peroxide solution, and the like. During operation of the dryer and sanitizer unit 10, air 58 entering the unit may be conditioned by the removable filter 64 to remove particulates and air-borne bacteria and/or viruses from the air. The filter housing 62 may be fixedly attached to the housing adjacent to air inlet vents 50, or may be removably attached to the housing adjacent to the air inlet vents 50.

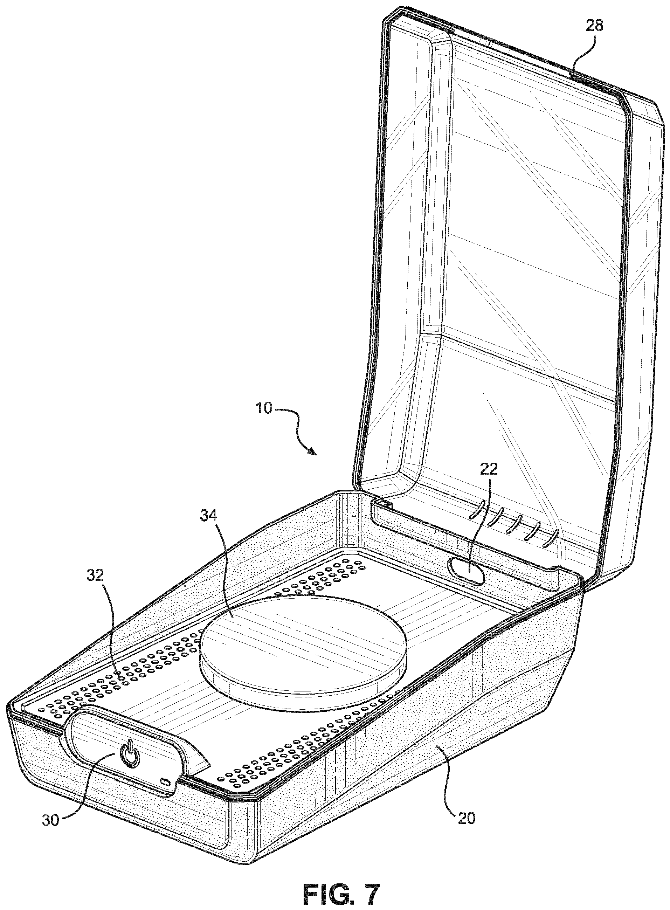

FIG. 7 illustrates another alternative embodiment, wherein the base 32 contains an inductive coil and associated circuitry below the platform 34 to provide wireless nearfield proximity charging for the electronic devices placed on base 32. In this embodiment, the rechargeable electronic devices may be set directly on the platform 34 above the inductive coil for charging.

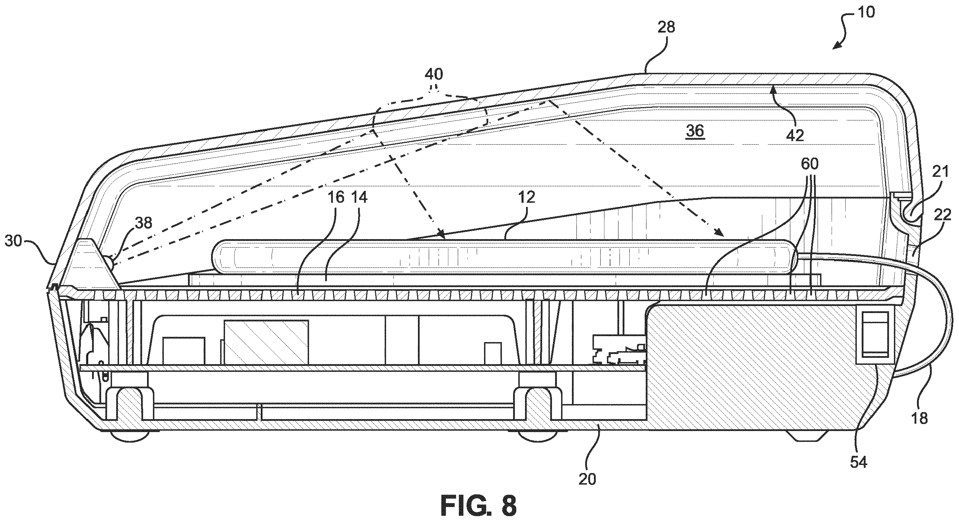

FIG. 8 is a cross-sectional view, not to scale, of the dryer and sanitizer unit 10 according to an embodiment of the invention. The dryer and sanitizer unit 10 is configured to include a drying chamber 36 provided by a lid 28 over a housing 20. The lid 28 may have a variety of shapes and sizes to accommodate a variety of rechargeable electronic devices of varying size. The unit 10 also includes one or more apertures 22 for passage of electric cords, such as the cord 18, out of the drying chamber 36. The apertures 22 are desirably located on housing 20 but could be provided on the lid 28 if desired.

The unit 10 also includes one or more disinfecting light sources 38 located within the drying chamber 36. The disinfecting light sources 38 are preferably UV-C lamps such as high intensity 50mm linear germicidal lamps operating at a wavelength of 253.7 nm and rated at 70uW/cm². The disinfecting light sources 38 may be placed in the drying chamber 36 at an angle to provide light at an angle ranging from about 15 to about 45 degrees so that the disinfecting light is reflected off of a reflective inside surface 42 of the lid 28 as illustrated by lines 40. The reflected light 40 from the reflective surface 42 of the lid is designed to illuminate the entire surface of the cell phone 12 or other rechargeable electronic device. Accordingly, the interior reflective surface 42 of the lid 28 may contain a mirror, reflective metal foil, or other UV reflective coating to reflect light from the disinfecting light sources 38 toward exposed surfaces of the rechargeable electronic devices.

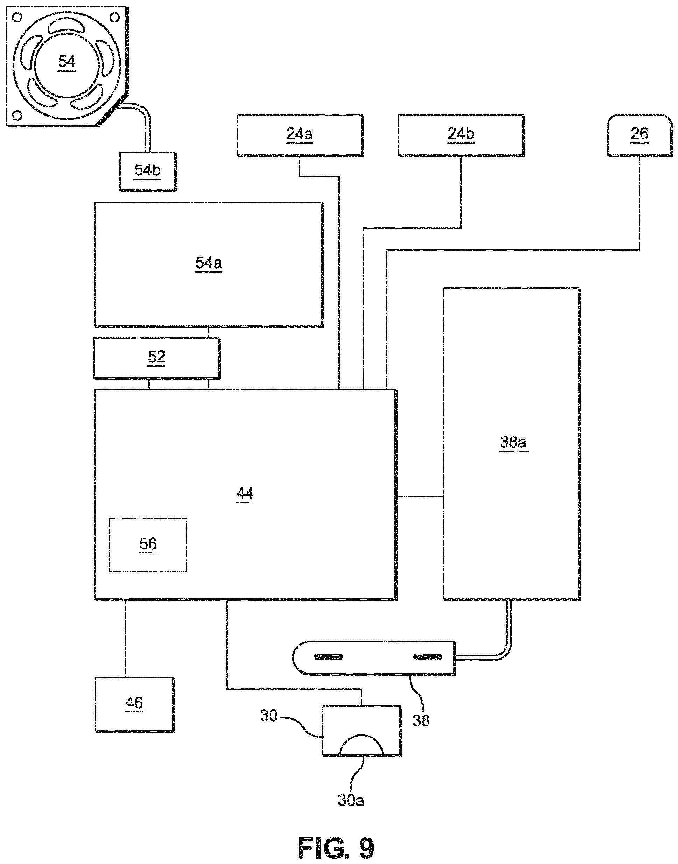

With additional reference to FIG. 9, various electronic components of an embodiment of the unit 10 are shown as located in the housing 20. The light sources 38 are preferably in electrical communication with a lamp control circuit 38a. Electrical power is controlled as by an on/off switch 30, such as a capacitive touch switch, with an LED indicator 30a to indicate that the unit 10 is on. Electrical power may be supplied to a charger 14 (shown in FIG. 2) by plugging the cord 14a into one or more power outlets 24a, 24b to which power is supplied by a microprocessor 44. The unit 10 preferably receives 5-volt direct current electrical power via one or more input power connectors 26, such as USB micro or USB-C connectors.

The lamp control circuit 38a may be programmed to operate the light sources 38 for a predetermined time sufficient to provide a 4-log theoretical kill rate of 99.99%. A desired operation time of the light sources 38 is from about 1.5 to about 3 minutes or longer to achieve this level of disinfection. In this regard, the lamp control circuit 38a is also in electrical communication with a microprocessor 44. The microprocessor 44 is configured to control the operation of the light circuit 38a. To enhance the effect of the light sources 38, one or more interior surfaces 42 of the lid 28 may be UV-reflective as described above.

In a preferred embodiment, the microprocessor 44 is also operably associated with a lid open detection circuit 46 in communication with both the lid 28 and the light sources 38. The microprocessor 44 is operable to turn off the light sources 38 if the lid open detection circuit 46 detects that the lid 28 is opened during operation of the disinfecting light sources 38.

The lid 28 preferably has a generally rectangular prism shape having multiple angled surfaces 42 as shown to reflect light from the disinfecting light sources 28 at multiple angles of incidence toward the rechargeable electronic devices. Air outlet vents 48 (FIG. 4) are included in the lid 28. The housing 20 includes air inlet vents 50 through which air enters the housing 20 to be heated so as to provide heated dry air into the drying chamber 36.

The heated dry air is preferably supplied at a temperature that fits the specification of the manufacturer of the batteries for the hearing aids. Generally speaking, however, it has been observed that damage can occur to some rechargeable batteries for certain rechargeable electronic devices if they are exposed to temperatures above about 85 degrees F (29 degrees C) for prolonged periods of time. The flow rate and flow time of the heated dry air is selected based on observation of parameters that provide optimal drying, and the microprocessor 44 is programmed to provide a desired air temperature, time and fan speed to provide the desired drying effect.

A heater 52 is also operably associated with the microprocessor 44. In addition, the microprocessor 44 is operably associated with a fan 54 connected to a fan circuit 54a via a fan power connector 54b to introduce the heated dry air into the drying chamber 36 through the inlet air vents 50 and to circulate the heated dry air in the drying chamber 36. The microprocessor 44 controls operation of the heater 52 and the fan 54 to provide a desired flow of the heated dry air into the drying chamber 36. The fan 54 preferably uses a magnetic bearing assembly, available from Sunon (www.sunon.com) under the name "MAGLEV Motor Fan" instead of ball bearing types of fan motors. This fan assembly allows the unit 10 to operate with less motor noise, as well as extended life, and a higher level of reliability.

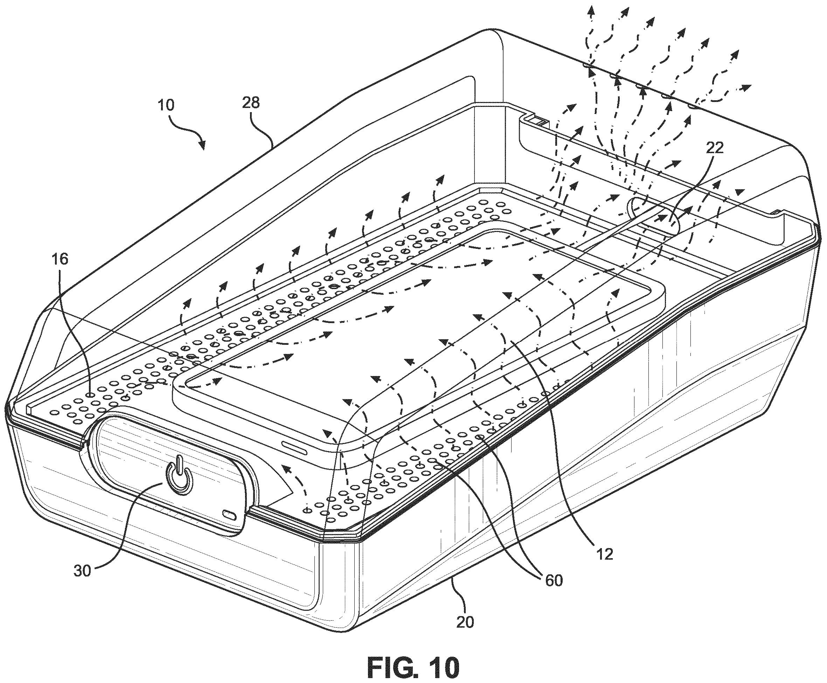

As illustrated in FIG. 10, the heated dry air may exit the drying chamber 36 via the outlet vents 48 in the lid 28. It is believed that operation of the unit 10 in a manner to provide a desirably controlled temperature in the drying chamber 36 in combination with a desired flow of moving air function to break the surface tension bond that water molecules have on surfaces of the rechargeable electronic devices, with the heated dry air circulating throughout the drying chamber 36 and exiting the drying chamber 36 through the outlet vents 48 serving to remove moisture from the drying chamber 36.

The microprocessor 44 includes a thermostat 56 configured for measurement of air temperature in the drying chamber 36. The microprocessor 44 is programmed to maintain the air temperature in the drying chamber 36, that is the temperature of the heated dry air, above a predetermined drying temperature and below a predetermined upper temperature corresponding to a temperature above which damage would occur to rechargeable batteries of the electronic devices in the drying chamber 36.

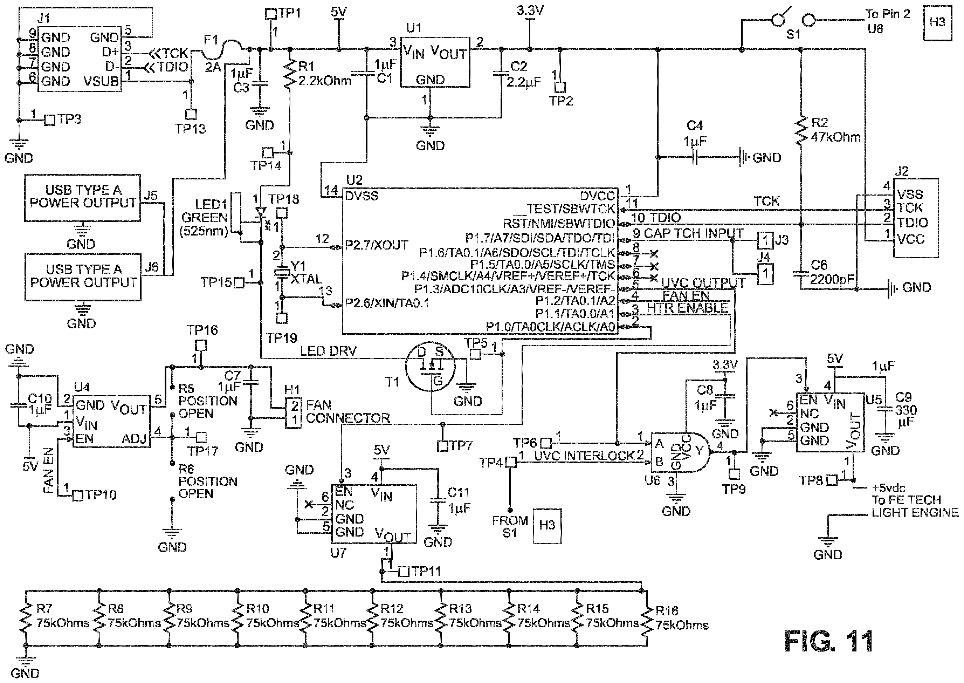

The microprocessor 44 is preferably a customizable digitally programmable microprocessor with non-volatile memory and the thermostat 56 is preferably an internal digital thermostat for controlling the temperature within the drying chamber 36 to a high level of thermal accuracy. The microprocessor 44 also controls the on/off times of the light sources 38 and then the heater 52 and the fan 54 to provide a desired treatment cycle and automatically turns off after the treatment cycle has completed or if the lid 28 has been opened. The cell phones 12 or other rechargeable electronic devices are recharged during the treatment cycle and may be left on the charger 14 following the treatment cycle if desired. In this regard, a treatment cycle is understood to include sanitization via the light source 38 followed by drying via the heated dry air. An illustrative schematic drawing of a circuit diagram for operation of the unit 10 is illustrated in FIG. 11.

During use of the unit 10, as depicted in FIGs. 8 and 10, a user may place a cell phone 12 inside the drying chamber 36, shut the lid 28, and press the on/off switch 30. The microprocessor 44 then initiates a sanitization cycle by turning on the light sources 38, to disinfect all exposed surfaces of the cell phone 12. After a pre-determined time has elapsed, the microprocessor 44 turns off the light sources 38 and initiates a drying cycle by turning on the heater 52, the fan 54 and the thermostat 56.

Next, as shown in FIGs. 8 and 10, air, indicated by arrows 58, is introduced to the drying chamber 36 through the vents 50 of the housing 20, and circulates within the drying chamber 36 through air inlet vents 50 in the housing 20 and a plurality of holes 60 in the base 16, and leaves the drying chamber 36 through the air outlet vents 48 in the lid 28. The circulating air is heated by heater 52 to provide heated dry air that functions to break the surface tension of water molecules on the surface of the rechargeable electronic device and draws the moisture away therefrom and out of the drying chamber 36 through the outlet vents 48 in the lid 28. Meanwhile, the microprocessor 44 monitors the temperature in the drying chamber using the thermostat 56 and maintains a pre-programmed temperature by controlling the output of the heater 52 and the speed of the fan 54. Precise control of the temperature of the circulating air is imperative to prevent overheating of the batteries of the rechargeable electronic device and/or the charger 14. At the conclusion of the drying phase of the treatment cycle, the microprocessor 44 turns off the heater 52 and the fan 54 and the rechargeable electronic device may be removed from the drying chamber 36.

To accommodate electronic devices that recharge using wireless nearfield proximity charging, an embodiment of the unit 10 (FIG. 7) includes in the base 32 an inductive coil and associated circuitry to provide the wireless nearfield proximity charging. In this embodiment, the rechargeable electronic devices may be set directly on the platform 34 of the base 32 above the inductive coil for charging.

The entire base 16, housing 20 and lid 28 may be configured of various colors, shapes, and dimensions depending on the device(s) being charged. The materials for the base, lid and housing may be made from a wide variety of metal and plastic materials in in one embodiment may be made of flame retardant acrylonitrile butadiene styrene materials.

The description of preferred embodiments for this disclosure has been presented for purposes of illustration and description. It is not intended to be exhaustive or to limit the disclosure to the precise form disclosed. Obvious modifications or variations are possible in light of the above teachings. The embodiments are chosen and described in an effort to provide the best illustrations of the principles of the disclosure and its practical application, and to thereby enable one of ordinary skill in the art to utilize the disclosure in various embodiments and with various modifications as are suited to the particular use contemplated. All such modifications and variations are within the scope of the disclosure as determined by the appended claims when interpreted in accordance with the breadth to which they are fairly, legally, and equitably entitled.

## Claims

1. A dryer and sanitizer unit, comprising:
a base upon which one or more rechargeable electronic devices may be placed;
a lid attached to the base that together with the base defines a drying chamber that encloses the one or more rechargeable electronic devices;
one or more disinfecting light sources disposed within the drying chamber;
one or more air inlets in gas flow communication with the drying chamber;
a heater configured to provide heated dry air from the one or more air inlets to the drying chamber;
a fan configured to circulate the heated dry air in the drying chamber;
a thermostat configured to measure air temperature in the drying chamber; and
a controller in electrical communication with the thermostat and the heater, the controller for controlling the heater to maintain the air temperature in the drying chamber above a predetermined drying temperature and below a predetermined upper temperature corresponding to a temperature above which rechargeable batteries associated with the rechargeable electronic devices are vulnerable to overheating.

2. The dryer and sanitizer unit of claim 1 further comprising one or more filters associated with the one or more air inlets.

3. The dryer and sanitizer unit of claim 2, wherein the one or more filters comprise a high-efficiency particulate air (HEPA) filter or a filter material impregnated with an essential oil.

4. The dryer and sanitizer unit of claim 1, wherein an interior surface of the lid is UV-reflective.

5. The dryer and sanitizer unit of claim 4 wherein the one or more disinfecting light sources are attached to the base at an angle to provide light reflected from the interior surface of the lid at an angle ranging from about 15 to about 45 degrees.

6. The dryer and sanitizer unit of claim 1, further comprising a lid open detection circuit operably associated with both the lid and the one or more disinfecting light sources, wherein the lid open detection circuit turns off the one or more disinfecting light sources if the lid is opened during operation of the one or more disinfecting light sources.

7. The dryer and sanitizer unit of claim 1, wherein the one or more rechargeable electronic devices comprise one or more hearing aids, cell phones, watches, headphones, or earphones.

8. The dryer and sanitizer unit of claim 1, wherein the one or more air inlets include air vents located in the lid to promote the flow of warm air over the rechargeable electronic devices and provide a path for the warm air to exit the drying chamber.

9. The dryer and sanitizer unit of claim 1, wherein the one or more disinfecting light sources are operated for a predetermined time sufficient to disinfect the rechargeable electronic devices within the drying chamber, after which the heater and fan are operated for a predetermined drying cycle to introduce the warm air into the drying chamber with or without the disinfecting light sources being operated.

10. The dryer and sanitizer unit of claim 1, further comprising a wireless nearfield proximity charger disposed in the base.

11. The dryer and sanitizer unit of claim 1 further comprising one or more passages in the lid or the base through which electric cords used in charging the one or more rechargeable electronic devices pass out of the drying chamber.

12. The dryer and sanitizer unit of claim 1 wherein the lid has a shape that is sufficient to accommodate the one or more rechargeable electronic devices and one or more electronic charger units for charging the one or more rechargeable electronic devices.

13. The dryer and sanitizer unit of claim 1 wherein the one or more disinfecting light sources comprise one or more UV-C light sources.
